# EUROPÄISCHE PATENTSCHRIFT

(11) **EP 1 575 378 B1**
(45) Veröffentlichungstag und Bekanntmachung des Hinweises auf die Patenterteilung: **11.06.2008**
(21) Anmeldenummer: 03817484.3
(22) Anmeldetag: 11.11.2003
(51) Int. Cl.: A23L 1/30, A61K 31/575

(54) **PULVERFÖRMIGE PHYTOSTEROL-FORMULIERUNGEN**
PULVERULENT PHYTOSTEROL FORMULATIONS
FORMULATIONS PULVERULENTES A BASE DE PHYTOSTEROLS

(30) Priorität: 13.11.2002 DE 10253111
(43) Veröffentlichungstag der Anmeldung: 21.09.2005
(73) Patentinhaber: BASF SE, 67056 Ludwigshafen (DE)
(72) Erfinder: AUWETER, Helmut, 67117 Limburgerhof (DE); BOHN, Heribert, 67319 Wattenheim (DE); HASSELWANDER, Oliver, 76829 Landau (DE); RUNGE, Frank, 67159 Friedelsheim (DE)
(86) Internationale Anmeldenummer: PCT/EP2003/012557
(87) Internationale Veröffentlichungsnummer: WO 2005/009144

(56) Entgegenhaltungen:
- WO-A-01/00046
- WO-A-01/32036
- WO-A-02/17892
- FR-A- 2 817 478
- US-A- 4 522 743
- US-A1- 2002 048 606
- US-B1- 6 391 370
- US-B1- 6 576 285
- SJOSTROM B ET AL: "A METHOD FOR THE PREPARATION OF SUBMICRON PARTICLES OF SPARINGLY WATER-SOLUBLE DRUGS BY PRECIPITATION IN OIL-IN-WATER EMULSIONS. II: INFLUENCE OF THE EMULSIFIER, THE SOLVENT, AND THE DRUG SUBSTANCE" JOURNAL OF PHARMACEUTICAL SCIENCES, AMERICAN PHARMACEUTICAL ASSOCIATION. WASHINGTON, US, Bd. 82, Nr. 6, 1. Juni 1993 (1993-06-01), Seiten 584-589, XP000367863 ISSN: 0022-3549

## Beschreibung

Die Erfindung betrifft Verfahren zur Herstellung von pulverförmigen Phytosterol-Formulierungen.

Als Phytosterole werden Sterole bezeichnet, die aus Pflanzen und Hefen isoliert werden. Die wichtigsten Vertreter dieser Stoffklasse sind z.B. Stigmasterol, Campesterol und β-Sitosterol sowie deren hydrierte Derivate wie Campestanol und β-Sitostanol. Phytosterole sind strukturell dem Cholesterol ähnlich. Da beispielsweise β-Sitosterol die Absorption von Cholesterol hemmt, wird es als Lipidsenker zur Prophylaxe von Arteriosklerose und Hyperlipidämie eingesetzt.

Zur Senkung des Cholesterinspiegels werden Phytosterole häufig als Zusatzstoffe in diätetischen Lebensmitteln wie z.B. für Margarine verwendet.

Phytosterole sind in Wasser unlöslich, während in Fetten und Ölen eine nur geringe Löslichkeit gefunden wird. Diese begrenzte Löslichkeit erschwert häufig die Anwendbarkeit der Phytosterole bei der Herstellung von Lebensmittelzubereitungen sowie von kosmetischen Produkten. Unzureichende Wirksamkeiten einerseits sowie eine schlechte Dispergierbarkeit in kosmetischen und Lebensmittelzubereitungen andererseits resultieren häufig aus den schlechten Löslichkeiten der Phytosterole.

Verschiedene Verfahren zur Herstellung Phytosterol-haltiger Formulierungen sind bereits bekannt. So beschreibt EP-A-0 289 636 Solubilisate von Phytosterolen in einer wässrigen Lösung von Polyhydroxyverbindungen oder Sucrose-Fettsäureestern.

Weitere flüssige Zubereitungen von Phytosterolen zusammen mit Solubilisatoren sind offenbart in US 3,865,939 und US 5,244,887.

EP-A-1 197 153 beschreibt wässrige Dispersionen oder Suspensionen von Phytosterolen in Gegenwart von nicht-sterolartigen Emulgatoren und deren Verwendung in Lebensmitteln, beispielweise in Brotaufstrichen.

WO 01/37681 betrifft wässrige Phytosterol-haltige Zusammensetzungen, erhältlich durch Homogenisieren von Phytosterolen in Wasser in Gegenwart eines wasserlöslichen Proteins, beispielsweise in Gegenwart von Casein und daraus hergestellte wasserdispergierbare Pulver.

WO 01/00046 A offenbart nanoskalige Phytosterole mit Teilchendurchmessern von 10 - 300 nm, welche nach einem Verfahren gewonnen werden, wobei die Ausgangsstoffe in einem geeigneten Lösungsmittel unter überkritischen oder nahekritischen Bedingungen gelöst werden, die fluide Mischung über eine Düse in ein Vakuum, ein Gas oder eine Flüssigkeit entspannt wird, und dabei das Lösungsmittel gleichzeitig verdampft wird.

US 6,391,371 offenbart Phytosterol-Dispersionen, die durch Mahlung in wässrigem Medium in Gegenwart eines Emulgators hergestellt werden.

Aufgabe der vorliegenden Erfindung war es, Verfahren zur Herstellung von Phytosterol-haltigen Formulierungen bereitzustellen, die sowohl in wässrige als auch in ölige Zubereitungen eingearbeitet werden können.

Diese Aufgabe wurde erfindungsgemäß gelöst durch ein Verfahren zur Herstellung von pulverförmigen Phytosterol-Formulierungen, enthaltend mindestens ein Phytosterol mit einer mittleren Teilchengröße im Bereich von 0,05 bis 1 µm, dadurch gekennzeichnet, dass mindestens ein Phytosterol in teilamorpher Form vorliegt, dadurch gekennzeichnet, dass man
a) ein oder mehrere Phytosterole in einem mit Wasser mischbaren, organischen Lösungsmittel oder einer Mischung aus Wasser und einem mit Wasser mischbaren, organischen Lösungsmittel bei Temperaturen im Bereich von 50°C bis 240°C, bevorzugt im Bereich von 100°C bis 200°C, besonders bevorzugt von 140°C bis 180°C löst,
b) die erhaltene Lösung mit einer wäßrigen molekulardispersen oder kolloiddispersen Lösung eines Schutzkolloids, ausgewählt aus der Gruppe, bestehend aus Pektin, Kasein, Natrium-Kaseinat, Gummi Arabicum, modifizierte Stärke und Fischgelatine mischt, wobei sich eine Mischungstemperatur von etwa 35°C bis 80°C einstellt und
c) die gebildete Dispersion für die Herstellung eines Trockenpulvers von dem Lösungsmittel und dem Wasser befreit und, gegebenenfalls in Gegenwart eines Überzugsmaterials, trocknet.

Als Phytosterole sind im Rahmen der Erfindung bevorzugt die drei Verbindungen Stigmasterol, Campesterol und β-Sitosterol sowie deren hydrierte Derivate Stigmastanol, Campestanol und β-Sitostanol gemeint. Besonders bevorzugt sind die durch Destillation aus Sojaöl gewonnenen Phytosterol Mischungen, die im wesentlichen aus Stigmasterol, Campesterol und β-Sitosterol bestehen.

Eine typische aus Pflanzenölen gewonnene Mischung dieser drei Phytosterole besteht aus ca. 40 bis 58 Gew.-% β-Sitosterol, 20 bis 30 Gew.-% Campesterol und 14 bis 22 Gew.-% Stigmasterol.

Je nach Art der verwendeten Lösungsmittel kann es sich bei der dispersen Phase im Schritt b) um feste Nanopartikel (Suspension) oder um Nanotröpfchen (Emulsion) handeln.

Die in der Stufe a) verwendeten wassermischbaren Lösungsmittel sind vor allem wassermischbare, thermisch stabile, flüchtige, nur Kohlenstoff, Wasserstoff und Sauerstoff enthaltenene Lösungsmittel wie Alkohole, Ether, Ester, Ketone und Acetale zu nennen. Zweckmäßig verwendet man solche Lösungsmittel, die mindestens zu 10 % wassermischbar sind, einen Siedepunkt unter 200°C aufweisen und/oder weniger als 10 Kohlenstoffe haben. Besonders bevorzugt werden Methanol, Ethanol, n-Propanol, Isopropanol, 1,2-Butandiol-1-methylether, 1,2-Propandiol-1-n-propylether, Tetrahydrofuran und/oder Aceton, ganz besonders bevorzugt n-Propanol, Isopropanol und/oder Aceton verwendet.

Unter Umständen kann es auch vorteilhaft sein, zusätzlich zu der Lösungsmittel-Phase ein physiologisch zugelassenes Öl wie beispielsweise Sesamöl, Maiskeimöl, Baumwollsaatöl, Sojabohnenöl oder Erdnußöl sowie Ester mittelkettiger pflanzlicher Fettsäuren in einer Konzentration von 0 bis 500 Gew.-%, vorzugsweise 10 bis 300 Gew.-%, besonders bevorzugt 20 bis 100 Gew.-%, bezogen auf das/die Phytosterol(e), zu geben, das dann gemeinsam mit den Wirkstoffen und den genannten Zusatzstoffen beim Mischen mit der wässrigen Phase extrem feinteilig ausgefällt wird.

Ganz besonders bevorzugt handelt es sich hierbei um ein Verfahren zur Herstellung von Trockenpulvern einer Mischung aus Stigmasterol, Campesterol und β-Sitosterol.

Da die Einwirkung hoher Temperaturen u. U. den gewünschten Gehalt an Phytosterolen herabsetzen kann, löst man das/die Phytosterol(e) möglichst rasch, beispielsweise im Sekundenbereich, z.B. in 0,1 bis 10 Sekunden, besonders bevorzugt in weniger als 1 Sekunde. Zur raschen Herstellung der molekulardispersen Lösung kann die Anwendung von erhöhtem Druck, z.B. im Bereich von 20 bar bis 80 bar, vorzugsweise 30 bis 60 bar, vorteilhaft sein.

Die so erhaltene molekulardisperse Lösung versetzt man anschließend direkt mit der gegebenenfalls gekühlten wäßrigen molekulardispersen oder kolloiddispersen Lösung des Schutzkolloids in der Weise, daß sich eine Mischungstemperatur von etwa 35°C bis 80°C einstellt.

Dabei wird die Lösungsmittelkomponente in die wäßrige Phase überführt und die hydrophobe Phase des/der Phytosterols/Phytosterole entsteht als disperse Phase.

Die mittlere Teilchengröße der nanopartikulären Teilchen in der wäßrigen Dispersion liegt je nach Art der Formulierungsmethode im Bereich von 0,01 bis 100 µm, bevorzugt im Bereich von 0,01 bis 10 µm, besonders bevorzugt im Bereich von 0,01 bis 2 µm, ganz besonders bevorzugt im Bereich von 0,05 bis 1 µm.

Hinsichtlich einer näheren Verfahrens- und Apparatebeschreibung zur oben genannten Dispergierung wird an dieser Stelle auf EP-B-0 065 193 Bezug genommen.

Die Überführung in ein Trockenpulver kann dabei u.a. durch Sprühtrocknung, Sprühkühlung, Gefriertrocknung oder Trocknung im Wirbelbett, gegebenenfalls auch in Gegenwart eines Überzugsmaterials erfolgen. Als Überzugsmittel eignen sich u.a. Maisstärke, Kieselsäure oder auch Tricalciumphosphat.

Gegenstand der Erfindung ist auch ein Verfahren zur Herstellung der oben genannten pulverförmigen Phytosterol-Formulierungen, dadurch gekennzeichnet, dass man mindestens ein Phytosterol in einem wäßrigen Medium in Gegenwart eines Schutzkolloids mahlt und die so erhaltene Phytosterol Suspension für die Herstellung eines Trockenpulvers trocknet, wobei man die Phytosterol Suspension nach dem Mahlen auf eine ausreichend hohe Temperatur erhitzt, um ein vollständiges oder teilweises Schmelzen der Phytosterole zu bewirken, und man diese Schmelze vor der Überführung in ein Trokkenpulver wieder abkühlt.

Die Mahlung kann dabei in an sich bekannter Weise z.B. mit einer Kugelmühle erfolgen. Dabei wird je nach verwendetem Mühlentyp so lange gemahlen, bis die Teilchen eine mittlere Partikelgröße von bevorzugt 0,2 bis 0,8 µm aufweisen.

Nähere Einzelheiten zur Mahlung und den dafür verwendeten Apparaturen finden sich u.a. in Ullmann's Encyclopedia of Industrial Chemistry, Sixth Edition, 1999, Electronic Release, Size Reduction, Kapitel 3.6.: Wet Grinding sowie in EP-A-0 498 824.

Bevorzugt wird dabei die Phytosterol Suspension nach dem Mahlen über einen Zeitraum von 0,05 bis 200 Sekunden, bevorzugt 0,2 bis 100 Sekunden auf eine Temperatur von 150 bis 200°C gehalten und vor der Überführung in ein Trockenpulver auf eine Temperatur von zwischen 20 und 80°C abgekühlt.

Je nach Trocknungsmethode weisen die Phytosterol-haltigen Trokkenpulver eine mittlere Partikelgröße von 100 bis 1000 µm bevorzugt von 200 bis 800 µm, besonders bevorzugt von 250 bis 600 µm auf. Diese Pulver stellen Agglomerate (Sekundärteilchen) der bereits eingangs mit einer mittleren Teilchengröße im Bereich von 0,01 bis 100 µm beschriebenen Primärteilchen dar.

Der Kristallinitätsgrad der Phytosterole in den nach dem erfindungsgemäßen Verfahren hergestellten Formulierungen läßt sich beispielsweise durch Röntgenbeugungsmessungen bestimmen und liegt im allgemeinen im Bereich kleiner 80 %, bevorzugt im Bereich von 30 bis 80 %, besonders bevorzugt im Bereich von 50 bis 80 %.

Eine weitere bevorzugte Ausführungsform der Phytosterol-Formulierungen ist dadurch gekennzeichnet, dass das Phytosterol in einer Schutzkolloid-Matrix eingebettet ist.

Geeignete Schutzkolloide sind sowohl elektrisch geladene Polymere (Polyelektrolyte) als auch neutrale Polymere. Typische Beispiele sind u.a. Gelatine wie Rinder-, Schweine- oder Fischgelatine, Stärke, modifizierte Stärke wie Octenylsuccinat Stärke, Dextrin, Pflanzenproteine wie Sojaproteine, die gegebenenfalls hydrolysiert sein können, Pektin, Guar gum, Xanthan, Gummi-Arabikum, Kasein, Natrium-Kaseinat, Ligninsulfonat oder Mischungen davon. Es können aber auch Methylcellulose, Carboxymethylcellulose, Hydroxypropylcellulose, Hydroxypropylmethylcellulose, Blätterschellack und Alginate eingesetzt werden. Weiterhin eignen sich Homo- und Copolymere auf Basis von neutralen, kationischen oder anionischen Monomeren wie z.B. Ethylenoxid, Propylenoxid, Acrylsäure, Maleinsäureanhydrid, Milchsäure, N-Vinylpyrrolidon, Vinylacetat, α- und β-Asparaginsäure. Bezüglich näherer Einzelheiten wird auf R.A. Morton, Fat Soluble Vitamins, Intern. Encyclopedia of Food and Nutrition, Bd.9, Pergamon Press 1970, S. 128-131, verwiesen.

Bevorzugte Schutzkolloide sind Verbindungen, ausgewählt aus der Gruppe, bestehend aus Gelatine wie Rinder-, Schweine- und Fischgelatine, Pflanzenproteine, Pektin, Kasein, Natrium-Kaseinat, Gummi Arabicum und modifizierte Stärke. Besonders bevorzugt eingesetzte Schutzkolloide sind Pektin, Kasein, Natrium-Kaseinat, Gummi Arabicum, modifizierte Stärke und/oder Fischgelatine.

Der Phytosterolgehalt in den nach den erfindungsgemäßen Verfahren hergestellten Formulierungen liegt im Bereich von 0,1 bis 80 Gew.-%, bevorzugt von 1 bis 50 Gew.-%, besonders bevorzugt von 3 bis 35 Gew.-%, ganz besonders bevorzugt im Bereich von 5 bis 25 Gew.-%, wobei sich die Gew.-Prozentangaben auf die Trockenmasse des Pulvers beziehen.

Die Menge an verwendeten Schutzkolloiden liegt im Bereich von 0,1 bis 80 Gew.-%, bevorzugt 5 bis 70 Gew.-%, besonders bevorzugt im Bereich von 10 bis 60 Gew.-%. Die Gewichtsprozentangaben beziehen sich auf die Trockenmasse der Phytosterol-Formulierung.

Zusätzlich können die Phytosterol-Formulierungen noch einen oder mehrere Weichmacher zur Erhöhung der mechanischen Stabilität der Pulver enthalten. Geeignete Weichmacher sind beispielsweise Zukker und Zuckeralkohole wie Saccharose, Glukose, Laktose, Invertzucker, Sorbit, Mannit, Xylit oder Glycerin. Die Weichmacher können in Mengen von 0,1 bis 70 Gew.-%, bevorzugt 10 bis 60 Gew.-%, besonders bevorzugt 20 bis 50 Gew.-%, bezogen auf die Trockenmasse der Phytosterol-Formulierungen enthalten sein.

Ferner können die Formulierungen einen oder mehrere niedermolekulare oberflächenaktive Verbindungen (Emulgatoren) in einer Konzentration von 0,01 bis 70 Gew.-%, vorzugsweise 0,1 bis 50 Gew.-%, besonders bevorzugt 0,5 bis 20 Gew.-%, bezogen auf die Trockenmasse der Phytosterol-Formulierungen enthalten. Als solche eignen sich vor allem amphiphile Verbindungen oder Gemische solcher Verbindungen. Grundsätzlich kommen alle lebensmittel- oder futtermitteltauglichen sowie pharmakologisch und dermatologisch unbedenklichen Tenside mit einem HLB-Wert von 5 bis 20 in Betracht. Als entsprechende oberflächenaktive Substanzen kommen beispielsweise in Betracht: Ester langkettiger Fettsäuren mit Ascorbinsäure, Mono- und Diglyceride von Fettsäuren und deren Oxyethylierungsprodukte, Ester von Monofettsäureglyceriden mit Essigsäure, Zitronensäure, Milchsäure oder Diacetylweinsäure, Polyglycerinfettsäureester wie z.B. das Monostearat des Triglycerins, Sorbitanfettsäureester, Propylenglykolfettsäureester und Lecithin. Bevorzugt wird Ascorbylpalmitat eingesetzt.

Weiterhin können die Formulierungen noch einen oder mehrere niedermolekulare Stabilisatoren wie Antioxidantien und/oder Konservierungsmittel enthalten. Geeignete Antioxidantien oder Konservierungsmittel sind beispielsweise α-Tocopherol, Ascorbinsäure, tert.-Butyl-hydroxytoluol, tert.-Butylhydroxyanisol, Lecithin, Ethoxyquin, Methylparaben, Propylparaben, Sorbinsäure oder Natriumbenzoat. Die Antioxidantien bzw. Konservierungsstoffe können in Mengen von 0,01 bis 50 Gew.-%, bevorzugt 0,1 bis 30 Gew.-%, besonders bevorzugt 0,5 bis 20 Gew.-%, ganz besonders bevorzugt 1 bis 10 Gew.-%, bezogen auf die Trockenmasse der Phytosterol-Formulierungen, vorliegen.

Neben den Phytosterolen können die nach den erfindungsgemäßen Verfahren hergestellten Formulierungen zusätzlich noch Carotinoide und Vitamine enthalten. Beispiele für Carotinoide sind u.a. β-Carotin, Bixin, Zeaxanthin, Cryptoxanthin, Citranaxanthin, Canthaxanthin, β-Apo-4-carotinal, β-Apo-8-carotinal, β-Apo-8-carotinsäureester, Astaxanthin, Lycopin oder Lutein, einzeln oder als Mischung.

Von den Vitaminen sind bevorzugt fettlösliche Vitamine wie Vitamin E, Vitamin E-Derivate z.B. Tocopherylacetat oder Tocopherylpalmitat sowie die K-Vitamine, Vitamin A und Derivate z.B. Vitamin A-Acetat, Vitamin A-Propionat oder Vitamin A-Palmitat, Vitamin D₂ und Vitamin D₃ und Mischungen zu verstehen. Die Bezeichnung Vitamin E steht in diesem Zusammenhang für natürliches oder synthetisches α-, β-, γ- oder δ-Tocopherol, bevorzugt für natürliches oder synthetisches α-Tocopherol sowie für Tocotrienol.

Die nach den erfindungsgemäßen Verfahren hergestellten Phytosterol-Formulierungen zeichnen sich u.a. dadurch aus, dass sie sowohl in öligen als auch in wässrigen Systemen, beispielsweise in Getränken gut dispergierbar sind.

Die Bestimmung der Partikelgröße, sowohl der Primär- als auch der Sekundärteilchen, erfolgt dabei mit Hilfe bekannter Meßmethoden u.a. über Fraunhofer Beugung sowie bei Partikeln kleiner 5 µm mit Hilfe dynamischer Lichtstreuung.

Die nach den erfindungsgemäßen Verfahren hergestellten Trockenpulver zeichnen sich u.a. dadurch aus, daß sie sich in wäßrigen Systemen unter Erzielung einer gleichmäßigen Feinverteilung des Wirkstoffes im Korngrößenbereich von 0,01 bis 1 µm problemlos wieder redispergieren lassen.

Die nach den erfindungsgemäßen Verfahren hergestellten Phytosterol-Formulierungen eignen sich u.a. als Zusatzstoff für Lebensmittelzubereitungen und Tierfuttermittel, als Mittel für die Herstellung pharmazeutischer und kosmetischer Zubereitungen sowie für die Herstellung von Nahrungsergänzungspräparaten im Human- und Tierbereich.

Ein typisches Einsatzgebiet im Lebensmittelbereich ist beispielsweise die Verwendung in Getränken, Milchprodukten wie Käse, Joghurt, Milchmixgetränke oder Milchspeiseeis sowie in Salatdressings, Saucen und Mayonnaisen aber auch in Wurstwaren und in Süßwaren.

Bevorzugt lassen sich die Suspensionen als Futtermittelzusatz in der Tierernährung einsetzen, insbesondere zum Auftragen bzw. Aufsprühen auf Futtermittelpellets.

Die Anwendung als Futtermittelzusatzstoff erfolgt insbesondere in Form flüssiger Zubereitungen, in denen die pulverförmigen Phytosterol-Formulierungen in einem Öl dispergiert vorliegen.

Als Öle kommen dabei in der Regel alle physiologisch unbedenklichen Öle - sowohl pflanzlichen als auch tierischen Ursprungs - in Frage, insbesondere solche Öle, die bei 20°C flüssig sind bzw. die in der Suspension bei 20°C allein oder zusammen mit anderen Ölen die flüssige Phase bilden. Bevorzugt zu nennen sind in diesem Zusammenhang Sonnenblumenöl, Palmöl, Sesamöl, Maiskeimöl, Baumwollsaatöl, Sojabohnenöl oder Erdnußöl, Ester mittelkettiger Triglyceride sowie außerdem Fischöle wie beispielsweise Makrelen-, Sprotten- oder Lachsöl. Für die Tierernährung besonders bevorzugt sind Fischöle, Maiskeimöl, Sonnenblumenöl und Erdnußöl.

Diese flüssigen Zubereitungen können beispielsweise durch direktes Aufsprühen auf Tierfutterpellets als sogenannte "post-pelleting-application" verabreicht werden.

Eine bevorzugte Ausführungsform des Sprühverfahrens besteht darin, daß man beispielsweise die Futtermittelpellets unter vermindertem Druck mit der öligen Suspension belädt.

Beispiele hierfür finden sich u.a. in GB-A-2 232 573 sowie in EP-A-0 556 883.

Typische Einsatzgebiete im Lebensmittelbereich sind beispielsweise die Vitaminierung von Getränken, Milchprodukten wie Joghurt, Milchmixgetränken oder Milchspeiseeis sowie von Puddingpulvern, Eiprodukten, Backmischungen und Süßwaren.

Im Kosmetikbereich können die öligen Suspensionen beispielsweise für Vitamin-haltige Körperpflegemittel beispielsweise in Form einer Creme, einer Lotion, als Lippenstifte oder Make-up verwendet werden.

Im Kosmetikbereich können die Phytosterol-Formulierungen beispielsweise als Emollient oder auch als wirkstoff in Hautpflegemittel verwendet werden.

Nahrungsergänzungsmittel, Tierfuttermittel, Lebensmittel sowie pharmazeutische und kosmetische Zubereitungen, können die oben beschriebenen Phytosterol-Formulierungen enthalten.

Unter Nahrungsergänzungspräparate sowie pharmazeutische Zubereitungen, die die nach den erfindungsgemäßen Verfahren hergestellte Phytosterol-Formulierung enthalten, sind u.a. Tabletten, Dragees sowie Hart- und Weichgelatinekapseln zu verstehen.

Als Lebensmittel sind beispielsweise Getränke, Milchprodukte wie Käse, Joghurt, Milchmixgetränke oder Milchspeiseeis sowie Salatdressings, Saucen oder Mayonnaisen Süßwaren und Wurstwaren gemeint, die die oben beschriebenen Phytosterol-Formulierungen enthalten.

Kosmetische Zubereitungen, die Phytosterol-Formulierungen enthalten können, sind beispielsweise topisch anwendbare Zubereitungen, insbesondere Hautpflegemittel und dekorative Körperpflegemittel wie Lippenstifte, Gesichts-Make-up in Form einer Creme, einer Lotion, eines Puders oder auch als Rouge.

Die pharmazeutischen Zubereitungen eignen sich zur Prophylaxe oder Therapie eines zu hohen Cholesterinspiegels.

In den nachfolgenden Beispielen werden die erfindungsgemäßen Verfahren zur Herstellung von Phytosterol-Formulierungen näher erläutert. Einzelheiten zu der in den Beispielen verwendeten Apparatur finden sich in EP-B-0 065 193.

### Beispiel 1

### Phytosterol Trockenpulver mit Natrium-Kaseinat

In einer Vorlage wurden 21 g Phytosterol (Fa. ADM, USA) und 2,1 g Ascorbylpalmitat in 360 g Aceton bei Raumtemperatur gelöst. In einer zweiten Vorlage wurden 35 g Na-Kaseinat und 35 g Saccharose in 4000 g vollentsalztem Wasser bei 70°C gelöst. Die Lösemittelphase, die auf 86,8°C eingestellt war, wurde anschließend mit einer Pumprate von 0,92 kg/h mit der wässrigen Phase bei Raumtemperatur mit einer Pumprate von 30,3 kg/h kontinuierlich vermischt. Die so entstandene Wirkstoffdispersion wurde an einem Rotationsverdampfer bei 65°C und einem Druck von 200 mbar von Aceton befreit und auf einen Feststoffgehalt von 11,5 Gew.-% aufkonzentriert. Die dabei entstandenen Wirkstoffteilchen wiesen eine Teilchengröße von 203 nm auf.

Anschließend wurde diese Dispersion auf einem Laborsprühturm sprühgetrocknet. Der Phytosterolgehalt in dem so erhaltenen Trokkenpulver betrug 26 Gew.-%. Das Trockenpulver ist in Wasser dispergierbar und nach Redispergierung ergab sich eine Teilchengröße von 1,08 µm.

### Beispiel 2

### Phytosterol Trockenpulver mit modifizierter Stärke

In einer Vorlage wurden 21 g Phytosterol (Fa. ADM, USA) und 2,1 g Ascorbylpalmitat in 360 g Aceton bei Raumtemperatur gelöst. In einer zweiten Vorlage wurden 35 g modifizierte Stärke (Emcap 12633, Fa. Cerestar, Krefeld) und 35 g Saccharose in 4000 g vollentsalztem Wasser bei 70°C gelöst. Die Lösemittelphase, die auf 94,9°C eingestellt war, wurde anschließend mit einer Pumprate von 2,61 kg/h mit der wässrigen Phase bei Raumtemperatur mit einer Pumprate von 30,0 kg/h kontinuierlich vermischt. Die so entstandene Wirkstoffdispersion wurde an einem Rotationsverdampfer bei 65°C und einem Druck von 200 mbar von Aceton befreit und auf einen Feststoffgehalt von 9,1 Gew.-% aufkonzentriert. Die dabei entstandenen Wirkstoffteilchen wiesen eine Teilchengröße von 264 nm auf.

Anschließend wurde diese Dispersion auf einem Laborsprühturm sprühgetrocknet. Der Phytosterolgehalt in dem so erhaltenen Trokkenpulver betrug 20,7 Gew.-%. Das Trockenpulver ist in Wasser dispergierbar und nach Redispergierung ergab sich eine Teilchengröße von 2,3 µm.

### Beispiel 3

### Phytosterol Trockenpulver mit modifizierter Stärke

40 g Phytosterol (Fa. ADM, USA), 6 g Ascorbylpalmitat und 40 g modifizierte Stärke (Capsul MKH, Fa. National Starch, Hamburg) wurden bei Raumtemperatur in 400 g vollentsalztem Wasser suspendiert. Der pH-Wert wurde anschließend mit 1 M NaOH auf pH 7,1 eingestellt. Diese Suspension wurde dann zusammen mit 2000 g Keramikkugeln (Zirkoniumoxid, Toray) des Durchmessers 1 mm in eine 1000 ml Glasflasche gegeben. Die Suspension wurde dann in dieser Glasflasche 8 Stunden auf einem Dispergiergerät (Red Devil) dispergiert. Die Wirkstoffteilchen hatten danach eine Größe von 585 nm.

Nach Abtrennung der Mahlkörper wurde 344 g Dispersion erhalten. In dieser wurden 28,3 g Saccharose gelöst. Anschließend wurde diese Dispersion auf einem Laborsprühturm sprühgetrocknet. Der Phytosterolgehalt in dem so erhaltenen Trockenpulver betrug 19,2 Gew.-%. Das Trockenpulver ist in Wasser dispergierbar und nach Redispergierung ergab sich eine Teilchengröße von 1,2 µm.

## Patentansprüche

1. Verfahren zur Herstellung von pulverförmigen Phytosterol-Formulierungen, enthaltend mindestens ein Phytosterol mit einer mittleren Teilchengröße von 0,05 bis 1 µm, **dadurch gekennzeichnet, dass** mindestens ein Phytosterol in teilamorpher Form vorliegt, **dadurch gekennzeichnet, dass** man
a) ein oder mehrere Phytosterole in einem mit Wasser mischbaren, organischen Lösungsmittel oder einer Mischung aus Wasser und einem mit Wasser mischbaren, organischen Lösungsmittel bei Temperaturen im Bereich von 50°C bis 240°C löst,
b) die erhaltene Lösung mit einer wäßrigen molekulardispersen oder kolloiddispersen Lösung eines Schutzkolloids, ausgewählt aus der Gruppe, bestehend aus Pektin, Kasein, Kaseinat, Gummi Arabicum, modifizierte Stärke und Fischgelatine mischt, wobei sich eine Mischungstemperatur von etwa 35°C bis 80°C einstellt und
c) die gebildete Dispersion für die Herstellung eines Trockenpulvers von dem Lösungsmittel und dem Wasser befreit und, gegebenenfalls in Gegenwart eines Überzugsmaterials, trocknet.

2. Verfahren zur Herstellung von pulverförmigen Phytosterol-Formulierungen, enthaltend mindestens ein Phytosterol mit einer mittleren Teilchengröße von 0,05 bis 1 µm, **dadurch gekennzeichnet, dass** mindestens ein Phytosterol in teilamorpher Form vorliegt, **dadurch gekennzeichnet, dass** man mindestens ein Phytosterol in einem wäßrigen Medium in Gegenwart eines Schutzkolloids mahlt und die so erhaltene Phytosterol Suspension für die Herstellung eines Trockenpulvers trocknet, wobei man die Phytosterol Suspension nach dem Mahlen auf eine ausreichend hohe Temperatur erhitzt, um ein vollständiges oder teilweises Schmelzen der Phytosterole zu bewirken, und man diese Schmelze vor der Überführung in ein Trockenpulver wieder abkühlt.

3. Verfahren nach Anspruch 2, **dadurch gekennzeichnet, dass** die Phytosterol Suspension nach dem Mahlen über einen Zeitraum von 0,05 bis 200 Sekunden auf eine Temperatur von 150 bis 200°C gehalten wird und vor der Überführung in ein Trockenpulver auf eine Temperatur von zwischen 20 und 80°C abgekühlt wird.

4. Verfahren nach einem der Ansprüche 1 bis 3, **dadurch gekennzeichnet, dass** in den pulverförmigen Phytosterol-Formulierungen das Phytosterol einen Kristallinitätsgrad im Bereich von 30 bis 80 %, bestimmt durch Röntgenbeugungsmessungen, aufweist.

5. Verfahren nach einem der Ansprüche 1 bis 4, **dadurch gekennzeichnet, dass** in den pulverförmigen Phytosterol-Formulierungen das Phytosterol in einer Schutzkolloid-Matrix eingebettet ist.

6. Verfahren nach einem der Ansprüche 1 bis 5, **dadurch gekennzeichnet, dass** die pulverförmigen Phytosterol-Formulierungen 0,1 bis 80 Gew.-% eines oder mehrerer Phytosterole enthalten, wobei sich die Gew.-Prozentangaben auf die Trockenmasse des Pulvers beziehen.

7. Verfahren nach Anspruch 6, **dadurch gekennzeichnet, dass** die pulverförmigen Phytosterol-Formulierungen 5 bis 70 Gew.-% eines oder mehrerer Schutzkolloide enthalten.

8. Verfahren nach einem der Ansprüche 6 oder 7, **dadurch gekennzeichnet, dass** die pulverförmigen Phytosterol-Formulierungen zusätzlich 0,1 bis 70 Gew.-% eines oder mehrerer Weichmacher enthalten.

9. Verfahren nach einem der Ansprüche 6 bis 8, **dadurch gekennzeichnet, dass** die pulverförmigen Phytosterol-Formulierungen zusätzlich 0,01 bis 70 Gew.-% eines oder mehrerer Emulgatoren enthalten.

10. Verfahren nach einem der Ansprüche 6 bis 9, **dadurch gekennzeichnet, dass** die pulverförmigen Phytosterol-Formulierungen zusätzlich 0,01 bis 50 Gew.-% eines oder mehrerer Antioxidantien und/oder Konservierungsmittel enthalten.

## Claims

1. A process for producing pulverulent phytosterol formulations comprising at least one phytosterol having a mean particle size of from 0.05 to 1 µm, wherein at least one phytosterol is present in partially amorphous form, wherein
a) one or more phytosterols is/are dissolved in a water-miscible organic solvent, or a mixture of water and a water-miscible organic solvent, at temperatures in the range from 50°C to 240°C,
b) the resultant solution is mixed with an aqueous molecular dispersion or colloidal dispersion of a protective colloid selected from the group consisting of pectin, casein, caseinate, gum arabic, modified starch and fish gelatin, a mixture temperature of from about 35°C to 80°C being established and
c) to produce a dry powder, the resulting dispersion is freed from the solvent and the water and is dried, if appropriate, in the presence of a coating maternal.

2. A process for producing pulverulent phytosterol formulations comprising at least one phytosterol having a mean particle size of from 0.05 to 1 µm, wherein at least one phytosterol is present in partially amorphous form, which comprises grinding at least one phytosterol in an aqueous medium in the presence of a protective colloid and drying the resultant Phytosterol suspension to produce a dry powder, the phytosterol suspension, after the grinding, being heated to a sufficiently high temperature to cause complete or partial melting of the phytosterols and this melt being cooled again before being converted into a dry powder.

3. The process according to claim 2, wherein the phytosterol suspension, after the grinding, is kept at a temperature of from 150 to 200°C for a period of from 0.05 to 200 seconds and is cooled to a temperature of from 20 to 80°C before conversion into a dry powder.

4. The process according to any of claims 1 to 3, wherein, in the pulverulent phytosterol formulations, the phytosterol has a degree of crystallinity in the range from 30 to 80%, determined by X-ray diffraction measurements.

5. The process according to any of claims 1 to 4, wherein, in the pulverulent phytosterol formulations, the phytosterol is embedded in a protective colloid matrix.

6. The process according to any of claims 1 to 5, wherein the pulverulent phytosterol formulations comprise from 0.1 to 80% by weight of one or more phytosterols, with the percentages by weight being based on the dry matter of the powder.

7. The process according to claim 6, wherein the pulverulent phytosterol formulations comprise from 5 to 70% by weight of one or more protective colloids.

8. The process according to either of claims 6 and 7, wherein the pulverulent phytosterol formulations additionally comprise from 0.1 to 70% by weight of one or more plasticizers.

9. The process according to any of claims 6 to 8, wherein the pulverulent phytosterol formulations additionally comprise from 0.01 to 70% by weight of one or more emulsifiers.

10. The process according to any of claims 6 to 9, wherein the pulverulent phytosterol formulations additionally comprise from 0.01 to 50% by weight of one or more antioxidants and/or preservatives.

## Revendications

1. Procédé de préparation de formulations de phytostérol sous forme de poudre, contenant au moins un phytostérol ayant une taille de particule moyenne de 0,05 à 1 µm, **caractérisé en ce qu'**au moins un phytostérol est présent sous une forme partiellement amorphe, **caractérisé en ce que** :
a) un ou plusieurs phytostérols sont dissous dans un solvant organique miscible avec l'eau ou dans un mélange d'eau et d'un solvant organique miscible avec l'eau, à des températures dans la plage de 50 à 240 °C,
b) la solution obtenue est mélangée avec une solution aqueuse à dispersion moléculaire ou à dispersion colloïdale d'un colloïde de protection, sélectionné parmi le groupe constitué de la pectine, la caséine, le caséinate, la gomme arabique, l'amidon modifié et la gélatine de poisson, une température de mélange d'environ 35 à 80 °C étant ajustée et
c) la dispersion formée est débarrassée du solvant et de l'eau pour la fabrication d'une poudre sèche, et, éventuellement en présence d'un matériau de revêtement, est séchée.

2. Procédé pour la préparation de formulations de phytostérol sous forme de poudre, contenant au moins un phytostérol ayant une taille de particule moyenne de 0,05 à 1 µm, **caractérisé en ce qu'**au moins un phytostérol est présent sous une forme partiellement amorphe, **caractérisé en ce qu'**au moins un phytostérol est broyé dans un milieu aqueux en présence d'un colloïde de protection et la suspension de phytostérol ainsi obtenue est séchée pour la fabrication d'une poudre sèche, la suspension de phytostérol étant chauffée après le broyage à une température suffisamment élevée pour provoquer une fusion complète ou partielle des phytostérols, et cette fonte est à nouveau refroidie avant la transformation en une poudre sèche.

3. Procédé selon la revendication 2, **caractérisé en ce que** la suspension de phytostérol est maintenue après le broyage à une température de 150 à 200 °C sur une période de temps de 0,05 à 200 secondes, et est refroidie avant la transformation en une poudre sèche à une température comprise entre 20 et 80 °C.

4. Procédé selon l'une quelconque des revendications 1 à 3, **caractérisé en ce que**, dans les formulations de phytostérol sous forme de poudre, le phytostérol présente un degré de cristallinité dans la plage de 30 à 80 %, déterminé grâce à des mesures par diffraction de rayon X.

5. Procédé selon l'une quelconque des revendications 1 à 4, **caractérisé en ce que**, dans les formulations de phytostérol sous forme de poudre, le phytostérol est plongé dans une matrice de colloïde de protection.

6. Procédé selon l'une quelconque des revendications 1 à 5, **caractérisé en ce que** les formulations de phytostérol sous forme de poudre contiennent 0,1 à 80 % en poids d'un ou plusieurs phytostérols, les indications de pourcentage en poids se rapportant à la masse sèche de la poudre.

7. Procédé selon la revendication 6, **caractérisé en ce que** les formulations de phytostérol sous forme de poudre contiennent 5 à 70 % en poids d'un ou plusieurs colloïdes de protection.

8. Procédé selon l'une des revendications 6 ou 7, **caractérisé en ce que** les formulations de phytostérols sous forme de poudre contiennent de manière supplémentaire 0,1 à 70 % en poids d'un ou plusieurs plastifiants.

9. Procédé selon l'une quelconque des revendications 6 à 8, **caractérisé en ce que** les formulations de phytostérol sous forme de poudre contiennent de manière supplémentaire 0,01 à 70 % en poids d'un ou plusieurs émulsifiants.

10. Procédé selon l'une quelconque des revendications 6 à 9, **caractérisé en ce que** les formulations de phytostérol sous forme de poudre contiennent de manière supplémentaire 0,01 à 50 % en poids d'un ou plusieurs antioxydants et/ou conservateurs.
